Europäisches Patentamt

European Patent Office

Office européen des brevets

(19)

(11) Veröffentlichungsnummer: **0 284 733 B1**

(12) **EUROPÄISCHE PATENTSCHRIFT**

(45) Veröffentlichungstag der Patentschrift: **11.03.92**

(51) Int. Cl.⁵: **A61B 6/06**

(21) Anmeldenummer: **88101639.8**

(22) Anmeldetag: **04.02.88**

(54) **Röntgenfernsehanlage zur Durchleuchtung eines Untersuchungsobjektes.**

(30) Priorität: **16.02.87 DE 3704859**

(43) Veröffentlichungstag der Anmeldung:
**05.10.88 Patentblatt 88/40**

(45) Bekanntmachung des Hinweises auf die
Patenterteilung:
**11.03.92 Patentblatt 92/11**

(84) Benannte Vertragsstaaten:
**DE FR GB NL**

(56) Entgegenhaltungen:
**EP-A- 0 139 941      EP-A- 0 176 282**
**EP-A- 0 188 783      DE-A- 3 030 332**
**DE-A- 3 147 128      DE-A- 3 436 866**
**DE-U- 8 226 150      US-A- 3 668 402**
**US-A- 3 934 151**

(73) Patentinhaber: **SIEMENS AKTIENGESELL-
SCHAFT
Wittelsbacherplatz 2
W-8000 München 2(DE)**

(72) Erfinder: **Hunold, Michael
Hinterm kreuzbrunnen
W-6682 Ottweiler(DE)**

## Beschreibung

Die Erfindung betrifft eine Röntgenfernsehanlage zur Durchleuchtung eines Untersuchungsobjektes mit einer Röntgenröhre, einer Primärstrahlenblende, einem Röntgenbildverstärker, einer Fernsehkamera und einem Sichtgerät, wobei zwischen der Fernsehkamera und dem Sichtgerät ein Bildspeicher und eine Bildverarbeitungseinrichtung vorgesehen sind, wodurch auf dem Sichtgerät wahlweise ein von der Fernsehkamera unmittelbar aufgenommenes oder ein in dem Bildspeicher gespeichertes Durchleuchtungsbild darstellbar ist, und wobei die Primärstrahlenblende zur Einblendung eines von der Röntgenröhre ausgehenden Röntgenstrahlenbündels verstellbare Blendenplatten aufweist.

Eine derartige Röntgenfernsehanlage ist in der Zeitschrift "Röntgenpraxis" 6/1981, Seiten 234 bis 246 beschrieben. Soll bei der bekannten Anlage ein Organ eines Patienten eingeblendet werden, sollen also die Blendenplatten der Primärstrahlenblende so verstellt werden, daß das durch sie eingeblendete Röntgenstrahlenbündel im wesentlichen nur das zu durchleuchtende Organ durchstrahlt, geschieht dies, indem der Patient durchleuchtet wird und die Blendenplatten, deren Schatten auf dem Sichtgerät sichtbar sind, in die erforderliche Position gebracht werden. Da dieser Vorgang unter Umständen eine erhebliche Zeit in Anspruch nehmen kann, besteht die Gefahr, daß der Patient einer Strahlendosis ausgesetzt wird, die gegenüber der zur eigentlichen Untersuchung erforderlichen nicht vernachlässigbar ist.

EP-A-0 139 941 beschreibt eine Vorrichtung bei der die Blendenplatten automatisch eingestellt werden entsprechend dem jeweiligen Bildfeld auf dem Lichtgerät, das mittels eines Lichtgriffels ausgewählt wird. Im Gegensatz zur Erfindung wird die eigentlich erreichte Stellung der Blendenplatten auf dem Sichtgerät nicht markiert.

Der Erfindung liegt die Aufgabe zugrunde, eine Röntgenfernsehanlage der eingangs genannten Art so auszubilden, daß die Einblendung eines Organs ohne nennenswerte zusätzliche Strahlenbelastung für den Patienten möglich ist.

Nach der Erfindung wird diese Aufgabe dadurch gelöst, daß ein einer Blendenplatte zugeordneter Weggeber vorgesehen ist, der ein der Stellung der Blendenplatte relativ zu dem Zentralstrahl des Röntgenstrahlenbündels entsprechendes Signal an die Bildverarbeitungseinrichtung gibt, die bei der Darstellung eines in dem Bildspeicher gespeicherten Durchleuchtungsbildes eine der Stellung der Blendenplatte entsprechende Marke in das Durchleuchtungsbild einblendet. Um ein Organ einblenden zu können, genügt es somit, den Patienten bei vollständig geöffneter Primärstrahlenblende kurzzeitig zu durchleuchten, das entsprechende Durchleuchtungsbild in dem Bildspeicher zu speichern und mittels der Bildverarbeitungseinrichtung auf dem Sichtgerät darzustellen. Die Einblendung des Organs kann dann anhand des gespeicherten Durchleuchtungsbildes erfolgen, in das mittels des Weggebers und der Bildverarbeitungseinrichtung die der Stellung der Blendenplatte entsprechende Marke eingeblendet ist. Während der Einblendung ist der Patient somit keiner Strahlenbelastung ausgesetzt.

Sofern sämtliche Blendenplatten der Primärstrahlenblende, wie beispielsweise im Falle einer Irisblende, synchron gegeneinander verstellbar sind, genügt es, wenn einer einzigen Blendenplatte ein Weggeber zugeordnet ist. Die die Position der übrigen Blendenplatten angebenden Marken können mittels einer geeignet ausgebildeten Bildverarbeitungseinrichtung oder einer zusätzlich vorhandenen, mit der Bildverarbeitunseinrichtung zusammenwirkenden Recheneinrichtung zusätzlich in das gespeicherte Durchleuchtungsbild eingeblendet werden. Sind sämtliche Blendenplatten der Primärstrahlenblende unabhängig voneinander verstellbar, ist nach einer Variante der Erfindung vorgesehen, daß jeder Blendenplatte ein Weggeber zugeordnet ist. Sind dagegen die Blendenplatten der Primärstrahlenblende wie im Falle der bekannten Rechteckblende paarweise synchron gegeneinander verstellbar, ist nach einer Ausführungsform der Erfindung vorgesehen, daß jeweils einer Blendenplatte eines Paares von Blendenplatten ein Weggeber zugeordnet ist und die Bildverarbeitungseinrichtung die der anderen Blendenplatte eines Paares entsprechende Marke erzeugt.

Um einer die Einblendung eines Organs vornehmenden Bedienperson einen Bildeindruck zu verschaffen, der dem bei der Einblendung eines Organs mittels der bekannten Röntgenfernsehanlage im wesentlichen entspricht, kann nach Varianten der Erfindung vorgesehen sein, daß die Marke durch eine Linie gebildet ist, die in ihrem Verlauf der Form der Blendenkante der zugehörigen Blendenplatte entspricht.

Nach einer Ausführungsform der Erfindung ist vorgesehen, daß der Weggeber unmittelbar mit der entsprechenden Blendenplatte verbunden ist. Sofern die Verstellung der Blendenplatten mototrisch erfolgt, kann vorgesehen sein, daß der Weggeber mit einem die entsprechende Blendenplatte verstellenden Motor gekuppelt ist. Falls die motorische Verstellung der Blendenplatten durch Schrittmotore erfolgt, kann aber auch vorgesehen sein, daß der Weggeber durch den Schrittmotor selbst und das Signal des Weggebers durch das Steuersignal für den Schrittmotor gebildet ist.

Ausführungsbeispiele der Erfindung sind in den beigefügten Zeichnungen dargestellt. Es zeigen:

| Fig. 1 | eine erfindungsgemäße Röntgenfernsehanlage in schematischer, perspektivischer Darstellung, |
| Fig. 2 und 3 | eine Einzelheit einer erfindungsgemäßen Röntgenfernsehanlage in schematischer Darstellung, und |
| Fig. 4 und 5 | ebenfalls eine Einzelheit einer erfindungsgemäßen Röntgenfernsehanlage in schematischer Darstellung. |

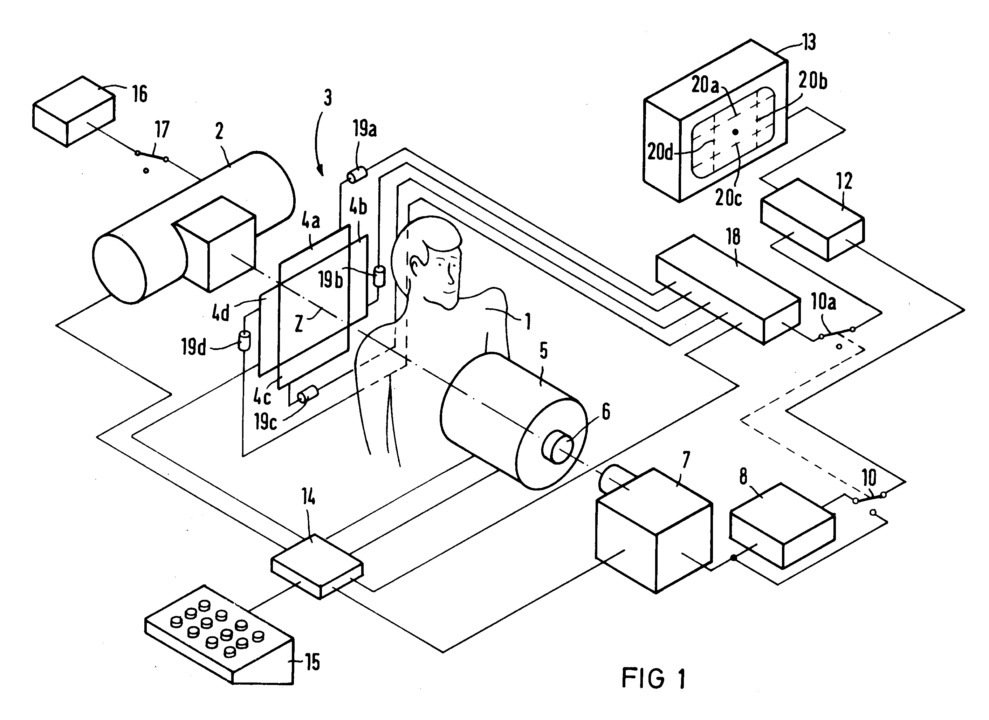

In Fig. 1 ist eine erfindungsgemäße Röntgenfernsehanlage zur Durchleuchung eines Patienten 1 dargestellt, die eine Röntgenröhre 2 aufweist, von der ein Röntgenstrahlenbündel ausgeht, von dem nur der Zentralstrahl Z in die Zeichnung eingetragen ist. Aus dem Röntgenstrahlenbündel wird mittels einer Primärstrahlenblende 3, die gegeneinander verstellbare Blendenplatten 4a, 4b, 4c und 4d aufweist, ein Strahlenkegel eingeblendet, der den Patienten 1 durchdringt und auf den Eingangsleuchtschirm eines Röntgenbildverstärkers 5 fällt. Das Bild des Ausgangsleuchtschirmes 6 des Röntgenbildverstärkers 5 wird mittels einer Fernsehkamera 7 aufgenommen, deren Signale einem Bildspeicher 8 zugeführt werden, von wo sie zu dem einen Pol 9 eines Umschalters 10 gelangen, dessen anderer Pol 11 unmittelbar mit der Fernsehkamera 7 verbunden ist. Mittels des Umschalters 10 kann wahlweise ein aus dem Bildspeicher 8 oder ein direkt von der Fernsehkamera 7 kommendes Fernsehsignal einer Bildverarbeitungseinrichtung 12 zugeführt werden, die ein zu ihr gelangendes Fernsehsignal zur Darstellung auf einem mit ihr verbundenen Sichtgerät 13 aufbereitet. Außerdem ist eine Steuerungseinheit 14 vorhanden, die mit einer Tastatur 15 verbunden ist und unter anderem den Bildspeicher 8 auf geeignete Betätigung der Tastatur 15 hin veranlassen kann, ein von der Fernsehkamera 7 geliefertes Durchleuchtungsbild zu speichern. Sie kann außerdem, ebenfalls auf eine geeignete Betätigung der Tastatur 15 hin, den Umschalter 10 dahingehend beeinflussen, daß entweder ein aus dem Bildspeicher 8 stammendes oder ein unmittelbar von der Fernsehkamera 7 aufgenommenes Durchleuchtungsbild auf dem Sichtgerät 13 dargestellt wird.

Mittels der Steuerungseinheit 14 und der Tastatur 15 ist es außerdem möglich, durch Betätigen eines zwischen einem die Röntgenröhre 2 versorgenden Generator 16 und der Röntgenröhre 2 vorgesehenen Schalters 17 die Röntgenröhre 2 wahlweise ein-bzw. auszuschalten. Dabei ist die Steuerungseinheit 14 derart ausgebildet, daß stets dann, wenn mittels des Umschalters 10 ein aus dem Bildspeicher 8 stammendes Durchleuchtungsbild auf dem Sichtgerät 13 dargestellt wird, die Röntgenröhre 2 von dem Generator 16 getrennt ist.

Mittels der Steuereinrichtung 14 und der Tatatur 15 besteht weiterhin die Möglichkeit, in nicht dargestellter Weise den Abstand zwischen der Röntgenröhre 2 und dem Eingangsleuchtschirm des Röntgenbildverstärkers 5 und den Abstand des Eingangsleuchtschirmes des Röntgenbildverstärkers 5 von dem Patienten 1 zu verändern, wobei, wie mittels der in Fig. 1 dargestellten, zwischen der Röntgenröhre 2 bzw. dem Röntgenbildverstärker 5 und der Steuerungseinheit 14 verlaufenden Leitungen schematisch angedeutet ist, eine Rückmeldung der aktuellen Position der Röntgenröhre 2 bzw. des Röntgenbildverstärkers 5 erfolgt. Der Röntgenbildverstärker 5 besitzt eine Elektronenoptik mit umschaltbarem Vergrößerungsfaktor, der mittels der Tastatur 15 und der Steuerungseinheit 14 zu beeinflussen ist, wobei eine Rückmeldung über den aktuellen Vergrößerungsfaktor der Elektronenoptik erfolgt, was durch eine weitere zwischen dem Röntgenbildverstärker 5 und der Steuerungseinheit 14 vorgesehene Leitung angedeutet ist. Auch der Vergrößerungsfaktor der Optik der Fernsehkamera 7 ist mittels der Steuerungseinheit 14 und der Tastatur 15 verstellbar. Auch hier erfolgt eine entsprechende Rückmeldung an die Steuerungseinheit 14, was durch eine zwischen der Fernsehkamera 7 und der Steuerungseinheit 14 verlaufende Leitung angedeutet ist. Aus den Werten für die aktuelle Position von Röntgenröhre 2 und Röntgenbildverstärker 5 relativ zu dem Patienten 1 sowie den Werten für die aktuellen Vergrößerungen des Röntgenbildverstärkers 5 und der Fernsehkamera 7 bildet die für diesen Zweck geeignet ausgebildete Steuerungseinheit 14 ein dem Abbildungsmaßstab eines auf dem Sichtgerät 13 dargestellten Durchleuchtungsbildes entsprechendes Signal und gibt dieses an eine Recheneinrichtung 18. Dieser werden außerdem Signale von mit den Blendenplatten 4a bis 4d unmittelbar verbundenen Weggebern 19a bis 19d zugeführt, die der Lage der jeweiligen Blendenplatte 4a bis 4d relativ zu dem Zentralstrahl Z des Röntgenstrahlenbündels entsprechen. Die Recheneinrichtung 18 ist derart ausgebildet, daß sie in der Lage ist, unter Berücksichtigung des von der Steuerungseinheit 14 stammenden Signales für den Abbildungsmaßstab aus den von den Weggebern 19a bis 19d gelieferten Signalen die Position der Blendenplatten 4a bis 4d in einem auf dem Sichtgerät 13 dargestellten Durchleuchtungsbild zu berechnen. Sobald auf dem Sichtgerät 13 ein aus dem Bildspeicher 8 stammendes Durchleuchtungsbild zur Darstellung kommt, werden der jeweiligen Position der Blendenplatten 4a bis 4d entsprechende Signale über einen mit dem Umschalter 10 gekoppelten Umschalter 10a der Bildverarbeitungseinrichtung 12 zugeführt, die unter Verarbeitung dieser Signale der Stellung der jeweiligen Blendenplatte 4a bis 4d entsprechende Marken 20a bis 20d in das aus dem Bildspeicher 8 stammende Durchleuchtungsbild einblendet. Werden während der Darstellung eines aus dem Bildspeicher 8 stammenden Durchleuchtungsbildes auf dem Sichtgerät 13 die Blendenplatten 4a bis 4d verstellt, hat dies eine Änderung der von den Weggebern 19a bis 19d zu der Recheneinrichtung 18 gelangenden Signale zur Folge, was sich in einer Änderung der von der Recheneinrichtung 18 an die Bildverarbeitungseinrichtung 12 gegebenen Signale und damit in einer der der Blendenplatten 4a bis 4d entsprechenden Bewegung der in das Durchleuchtungsbild eingeblendeten Marken 20a bis 20d auf dem Sichtgerät 13 auswirkt, die als in ihrem Verlauf der Form der Blendenkanten der Blendenplatten 4a bis 4d entsprechende strichlierte Linien auf dem Sichtgerät 13 dargestellt werden.

Soll mit der erfindungsgemäßen Röntgenfernsehanlage ein Organ des Patienten 1 eingeblendet werden, wird so vorgegangen, daß der Patient zunächst, z.B. mittels eines in Fig. 1 nicht dargestellten Lichtvisiers, bei ausgeschalteter Röntgenröhre 2 relativ zu dem Zentralstrahl Z des Röntgenstrahlenbündels so positioniert wird, daß dieser durch das zu untersuchende Organ verläuft. Anschließend wird der Patient 1 kurzzeitig durchleuchtet und das entsprechende Durchleuchtungsbild in dem Bildspeicher 8 gespeichert und mittels der Bildverarbeitungseinrichtung 12 auf dem Sichtgerät 13 dargestellt, wobei die Röntgenröhre 2 abgeschaltet ist. Die Blendenplatten 4a bis 4d, deren Position relativ zu dem Zentralstrahl Z auf dem Sichtgerät 13 durch die linienförmigen Marken 20a bis 20d angezeigt wird, werden nun gegeneinander verstellt, bis das zu untersuchende Organ in der gewünschten Weise eingeblendet ist, wobei dieser Vorgang jederzeit auf dem Sichtgerät 13 verfolgt werden kann. Nach erfolgter Einblendung des zu untersuchenden Organs kann die eigentliche Untersuchung erfolgen. Zu diesem Zweck wird durch entsprechendes Betätigen der Tastatur 15 die Röntgenröhre 2 eingeschaltet und der Umschalter 10 in eine solche Stellung gebracht, daß das von der Fernsehkamera 7 vom Ausgangsleuchtschirm 6 des Röntgenbildverstärkers 5 aufgenommene Durchleuchtungsbild mittels der Bildverarbeitungseinrichtung 12 unmittelbar auf dem Sichtgerät 13 angezeigt wird. Dabei befindet sich der Umschalter 10a in einer solchen Stellung, daß die Einblendung der Marken 20a bis 20d in das Durchleuchtungsbild unterbleibt, da die Schatten der Blendenplatten 4a bis 4d auf dem Sichtgerät 13 ohnehin sichtbar sind.

Es wird somit deutlich, daß mittels der Erfindung die Einblendung eines zu untersuchenden Organs praktisch ohne Strahlenbelastung des Patienten erfolgen kann. Sofern im Falle einer erfindungsgemäßen Röntgenfernsehanlage der Abbildungsmaßstab im wesentlichen, d.h., abgesehen von den durch die Lage des zu untersuchenden Organs innerhalb des Patienten gegebenen Schwankungen, fest ist, kann die Recheneinrichtung 18 entfallen. Die Signale der Weggeber 19a bis 19d können der Bildverarbeitungseinrichtung 12 dann unmittelbar zugeführt werden. Die Recheneinrichtung 18 muß übrigens nicht als separate Einrichtung ausgeführt sein, sondern kann auch Teil der Bildverarbeitungseinrichtung 12 oder der Steuerungseinheit 14 sein.

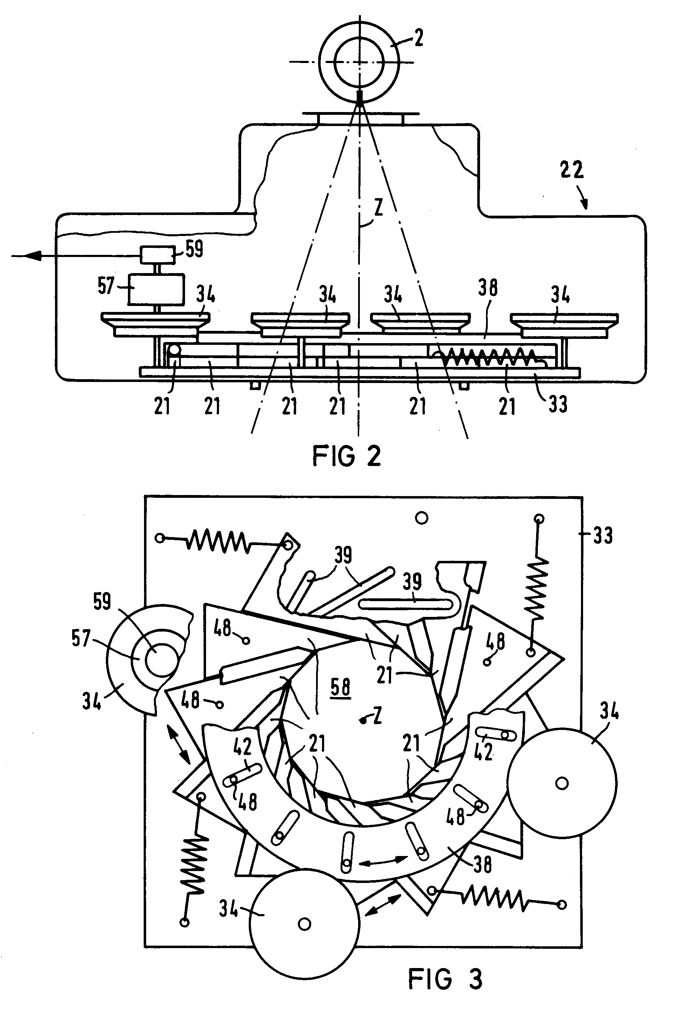

In den Fig. 2 und 3 ist eine Einzelheit, nämlich die Röntgenröhre 2 und die Primärstrahlenblende 22, einer erfindungsgemäßen Röntgenfernsehanlage dargestellt. Zur Einblendung einer Strahlenpyramide annähernd kreisförmigen Querschnittes weist die Primärstrahlenblende 22 dreieckige Blendenplatten 21 auf, die zwischen einer Tragplatte 33 und einem über der Tragplatte 33 an Führungsrollen 34 um den Zentralstrahl Z eines von einer Röntgenröhre 2 ausgehenden Röntgenstrahlenbündels drehbar gelagerten Stellring 38 gehaltert sind. In der Tragplatte 33 sind tangentiale Führungsnuten 39 und in dem Stellring 38 radiale Führungsnuten 42 angebracht, in die an den Blendenplatten 21 angebrachte Bolzen 48 eingreifen. In Fig. 3 sind die in der Tragplatte 33 vorgesehenen Führungsnuten 39 und die in dem Stellring 38 angebrachten Führungsnuten 42 sowie die Bolzen 48 sichtbar. Eine der Führungsrollen 34 ist mit einem Motor 57 verbunden, so daß der Stellring 38 bei Betätigen des Motors 57 über die angetriebene Führungsrolle 34 mitgenommen wird. Da die Blendenplatten 21 jede für sich mit ihrem Bolzen 48 einerseits in eine radiale Führungsnut 42 des Stellringes 38 und andererseits in eine dazu senkrechte Führungsnut 39 der Tragplatte 33 eingreifen, werden sie beim Drehen des Stellringes 38 mittels des Motors 57 relativ zu der Tragplatte 33 tangential zu der Blendenöffnung 58 synchron verschoben. Beim Verdrehen des Stellringes 38 in der Fig. 3 nach links verkleinert sich die Blendenöffnung 58, während sie sich beim Verdrehen des Stellringes 38 nach rechts vergrößert.

Mit dem Motor 57 ist ein Weggeber 59 verbunden, der ein der jeweiligen Stellung der Blendenplatten 21 relativ zu dem Zentralstrahl Z entsprechendes Signal liefert, das zur Einblendung von den Stellungen der Blendenplatten 21 relativ zu dem Zentralstrahl Z entsprechenden Marken in ein Durchleuchtungsbild dient und über die Leitung 23 zur Bildverarbeitungseinrichtung (nicht dargestellt) gelangt. Dabei genügt infolge des Umstandes, daß sämtliche Blendenplatten 21 synchron gegeneinander verstellbar sind, ein einziger Weggeber 59, sofern die Bildverarbeitungseinrichtung derart ausgebildet ist, daß sie ausgehend von dem Signal des Weggebers 59 eine der Anzahl und Lage der Blendenplatten 21 entsprechende Anzahl von Marken in ein gespeichertes Durchleuchtungsbild einblendet.

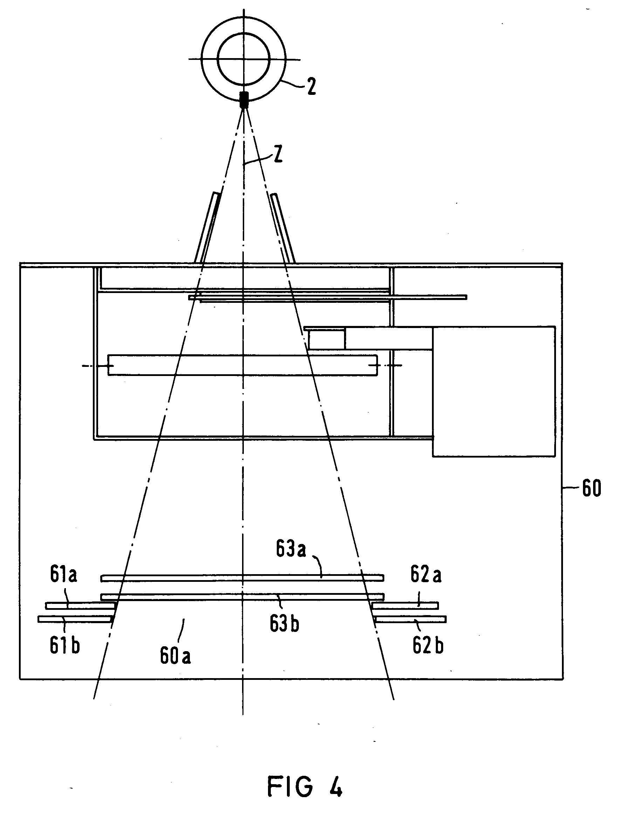

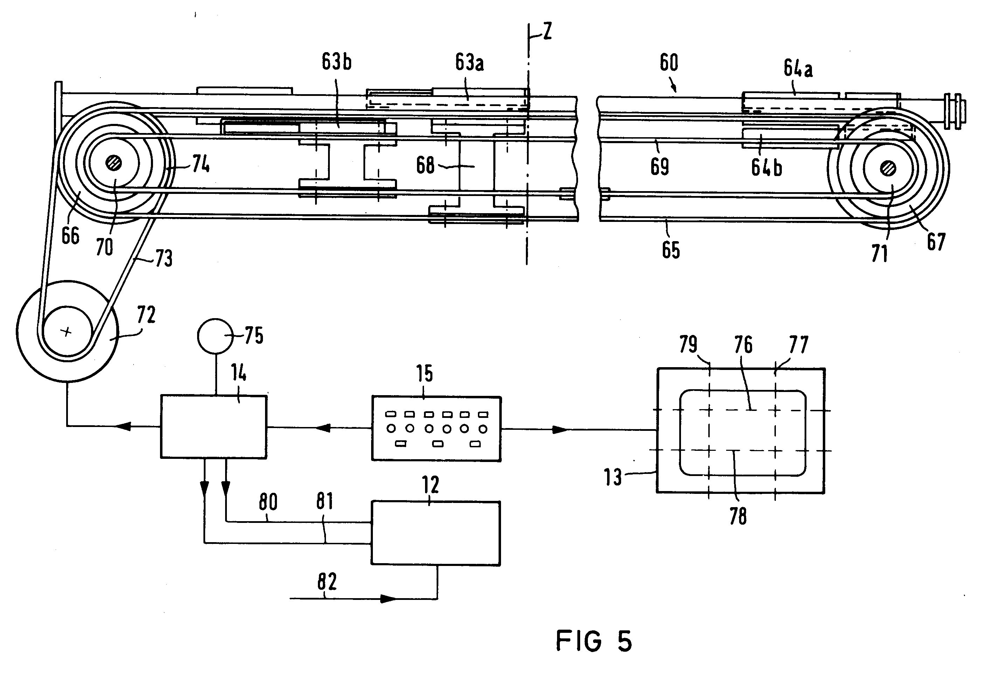

Auch in den Fig. 4 und 5 sind von einer erfindungsgemäßen Röntgenfernsehanlage im wesentlichen wieder nur die Röntgenröhre 2 und eine Primärstrahlenblende 60 mit einer rechteckigen Blendenöffnung 60a zur Einblendung einer rechteckigen Strahlenpyramide aus einem von der Röntgenröhre 2 ausgehenden Röntgenstrahlenbündel mit dem Zentralstrahl Z dargestellt. Die Primärstrahlenblende besitzt insgesamt acht Blendenplatten 61a, 61b, 62a, 62b, 63a, 63b, 64a, 64b, von denen in Fig. 4 die Blendenplatten 64a und 64b nicht sichtbar sind.

Die Blendenplatten 61a und 62a sowie 63a und 64a sind jeweils paarweise synchron gegeneinander verstellbar und begrenzen mit ihren dem Zentralstrahl Z des Röntgenstrahlenbündels zugewandten Kanten die Blendenöffnung 60a. Die Blendenplatten 61b, 62b sowie 63b, 64 folgen der Bewegung der Blendenplatten 61a, 62a bzw. 63a, 64a derart, daß sie sich stets mit diesen überlappen, so daß die an deren dem Zentralstrahl Z abgewandten Kanten etwa vorbeitretende Röntgenstrahlung ausgeblendet wird.

Der Mechanismus, der eine solche Bewegung der Blendenplatten 61a bis 64b bewirkt, ist am Beispiel der Blendenplatten 63a bis 64b in Fig. 5 dargestellt. Die Blendenplatten 63a und 64a sind an einem Zahnriemen 65 befestigt, der um zwei Räder 66, 67 geführt ist. Damit sich die Blendenplatten 63a und 64a synchron gegenläufig zueinander bewegen, ist die Blendenplatte 63a mit dem unteren Teil des Zahnriemens 65 mit Hilfe eines Zwischenstückes 68 verbunden, während die Blendenplatte 64a unmittelbar mit dem oberen Teil des Zahnriemens 65 verbunden ist. Die Blendenplatten 63b und 64b sind an einem zweiten Zahnriemen 69, der um Räder 70, 71 geführt ist, in analoger Weise angebracht. Die Räder 66, 70 und 67, 71 sitzen jeweils drehfest miteinander verbunden auf einer gemeinsamen Achse und haben unterschiedliche Durchmesser. Da die Räder 70, 71 einen kleineren Durchmesser aufweisen, legen die Blendenplatten 63b und 64b bei einer Drehung der Räder 66, 70; 67, 71 um einen bestimmten Winkel einen kleineren Weg zurück als die Blendenplatten 63a und 64a. Der Durchmesser der Räder 66, 67 ist um ein solches Maß größer als der der Räder 70, 71, daß der Stellweg für die Blendenplatten 63a, 64a um so viel größer als der Stellweg der Blendenplatten 63b, 64b ist, daß sich die Blendenplatten 63a und 63b bzw. 64a und 64b in jeder Blendenstellung überlappen.

In der linken Hälfte der Fig. 5 ist übrigens die Primärstrahlenblende 60 in vollständig geschlossenem und in der rechten Hälfte der Fig. 5 in vollständig geöffnetem Zustand dargestellt.

Die Verstellung der Blendenplatten 63a bis 64b erfolgt mittels eines Schrittmotors 72, der über einen Zahnriemen 73 ein mit den Rädern 66, 70 auf einer gemeinsamen Achse sitzendes Rad 74 antreibt.

Zur Verstellung der Blendenplatten 61a bis 62b ist ein entsprechender Mechanismus vorgesehen, von dem in Fig. 5 nur schematisch der zum Antrieb dieser Blendenplatten dienende Schrittmotor 75 dargestellt ist. Die Schrittmotore 72 und 75 sind mit der Steuereinrichtung 14 verbunden und können durch Betätigen der Tastatur 15 zum Einstellen einer gewünschten Blendenöffnung 60a aktiviert werden.

Um auch mit der Röntgenfernsehanlage nach den Fig. 4 und 5 die Einblendung eines Organs eines Patienten in der zuvor beschriebenen Weise durchführen zu können, gelangen von der Steuereinheit 14 die Steuersignale für die Schrittmotore 72 und 75, die deren aktuellen Drehwinkeln und damit der Lage der Blendenplatten 61a, 62a, 63a und 64a relativ zu dem Zentralstrahl Z des Röntgenstrahlenbündels entsprechen, über die Leitungen 80, 81 an die Bildverarbeitungseinrichtung 12, die entsprechende Marken 76 bis 79 in ein über die Leitung 82 von dem nicht gezeigten Bildspeicher ankommendes und auf dem Sichtgerät 13 dargestelltes Durchleuchtungsbild einblendet.

Im Falle der Röntgenfernsehanlage nach den Fig. 4 und 5 sind die Weggeber somit durch die zur Verstellung der Blendenplatten 61a bis 64b vorgesehenen Schrittmotore 72 und 75 gebildet, wobei die Signale der Weggeber durch die Steuersignale für die Schrittmotore 72 und 75 gebildet sind. Da im Falle der Röntgenfernsehanlage nach den Fig. 4 und 5 die Blendenplatten 61a und 62a bzw. 63a und 64a synchron paarweise gegeneinander verstellbar sind, ist für jedes Paar von Blendenplatten 61a, 62a und 63a, 64a jeweils nur ein Weggeber erforderlich, d.h. zusätzlich zu den Schrittmotoren 72 und 75 sind keine weiteren Weggeber erforderlich, da die Bildverarbeitungseinrichtung 12 derart ausgebildet ist, daß sie ausgehend von den Steuersignalen der Schrittmotore 72 und 75 jeweils zwei symmetrisch zum Zentralstrahl Z angeordnete Marken 76, 78 bzw. 77, 79 erzeugt und in das Durchleuchtungsbild einblendet.

Die Recheneinrichtung 18 kann unter Berücksichtigung der in der jeweiligen erfindungsgemäßen Röntgenfernsehanlage vorliegenden Verhältnisse sowohl als Digital- als auch als Analog-Rechner ausgeführt sein.

Die im Falle der erfindungsgemäßen Röntgenfernsehanlagen nach der Fig. 1 bzw. den Fig. 2 und 3 vorgesehenen Weggeber 19a bis 19d bzw.

59 können beispielsweise als Potentiometer ausgeführt sein. Es können aber auch induktiv, kapazitiv oder optisch wirkende Weggeber Verwendung finden.

**Patentansprüche**

1. Röntgenfernsehanlage zur Durchleuchtung eines Untersuchungsobjektes (1) mit einer Röntgenröhre (2), einer Primärstrahlenblende (3, 21, 60), einem Röntgenbildverstärker (5), einer Fernsehkamera (7) und einem Sichtgerät (13), wobei zwischen der Fernsehkamera (7) und dem Sichtgerät (13) ein Bildspeicher (8) und eine Bildverarbeitungseinrichtung (12) vorgesehen sind, wodurch auf dem Sichtgerät (13) wahlweise ein von der Fernsehkamera (7) unmittelbar aufgenommenes oder ein in dem Bildspeicher (8) gespeichertes Durchleuchtungsbild darstellbar ist, und wobei die Primärstrahlenblende (3, 22, 60) zur Einblendung eines von der Röntgenröhre (2) ausgehenden Röntgenstrahlenbündels verstellbare Blendenplatten (4a, 4b, 4c, 4d; 21; 61a, 62a, 63a, 64a) aufweist, **dadurch gekennzeichnet**, daß ein einer Blendenplatte (4a, 4b, 4c, 4d; 21; 61a, 62a, 63a, 64a) zugeordneter Weggeber (19a, 19b, 19c, 19d; 59; 72, 75) vorgesehen ist, der ein der Stellung der Blendenplatte (4a, 4b, 4c, 4d; 21; 61a, 62a, 63a, 64a) relativ zu dem Zentralstrahl (Z) des Röntgenstrahlenbündels entsprechendes Signal an die Bildverarbeitungseinrichtung (12) gibt, die bei der Darstellung eines in dem Bildspeicher (8) gespeicherten Durchleuchtungsbildes eine der Stellung der Blendenplatte (4a, 4b, 4c, 4d; 21; 61a, 62a, 63a, 64a) entsprechende Marke (20a, 20b, 20c, 20d; 76, 77, 78, 79) in das Durchleuchtungsbild einblendet.

2. Röntgenfernsehanlage nach Anspruch 1, **dadurch gekennzeichnet**, daß jeder Blendenplatte (4a, 4b, 4c, 4d) ein Weggeber (19a, 19b, 19c, 19d) zugeordnet ist.

3. Röntgenfernsehanlage nach Anspruch 1, mit einer Primärstrahlenblende (60) mit paarweise synchron gegeneinander verstellbaren Blendenplatten (61a, 62a bzw. 63a, 64a), **dadurch gekennzeichnet**, daß jeweils einer Blendenplatte (61a bzw. 63a) eines Paares von Blendenplatten (61a, 62a bzw. 63a, 64a) ein Weggeber (75 bzw. 72) zugeordnet ist und die Bildverarbeitungseinrichtung (12), die der anderen Blendenplatte (62a bzw. 64a) eines Paares entsprechende Marke (78 bzw. 79) erzeugt.

4. Röntgenfernsehanlage nach einem der Ansprüche 1 bis 3, **dadurch gekennzeichnet**, daß die Marke (20a, 20b, 20c, 20d; 76, 77, 78, 79) jeweils durch eine Linie gebildet ist.

5. Röntgenfernsehanlage nach Anspruch 4, **dadurch gekennzeichnet**, daß die Marke (20a, 20b, 20c, 20d; 76, 77, 78, 79) in ihrem Verlauf der Form der Blendenkante der entsprechenden Blendenplatte (4a, 4b, 4c, 4d; 61a, 61b, 61c, 61d) entspricht.

6. Röntgenfernsehanlage nach einem der Ansprüche 1 bis 5, **dadurch gekennzeichnet**, daß der Weggeber (19a, 19b, 19c, 19d) unmittelbar mit der entsprechenden Blendenplatte (4a, 4b, 4c, 4d) verbunden ist.

7. Röntgenfernsehanlage nach einem der Ansprüche 1 bis 5, **dadurch gekennzeichnet**, daß der Weggeber (59) mit einem die entsprechende Blendenplatte (21) verstellenden Motor (27) gekuppelt ist.

8. Röntgenfernsehanlage nach einem der Ansprüche 1 bis 5, **dadurch gekennzeichnet**, daß der Weggeber durch einen zur Verstellung der entsprechenden Blendenplatte (61a, 62a; 63a, 64a) vorgesehenen Schrittmotor (75, 72) und das Signal des Weggebers durch das Steuersignal für den Schrittmotor (75, 72) gebildet ist.

**Claims**

1. X-ray television installation for the fluoroscopy of an object to be examined (1) having an X-ray tube (2), a primary radiation diaphragm (3, 21, 60), an X-ray image intensifier (5), a television camera (7) and a display unit (13), whereby between the television camera (7) and the display unit (13) there are provided an image memory (8) and an image-processing device (12), whereby on the display unit (13) there can be represented selectively a fluoroscopic image, directly taken by the television camera (7), or one stored in the image memory (8), and whereby the primary radiation diaphragm (3, 22, 60) has adjustable diaphragm plates (4a, 4b, 4c, 4d; 21; 61a, 62a, 63a, 64a) for gating an X-ray beam emanating from the X-ray tube (2), characterized in that a position encoder (19a, 19b, 19c, 19d; 59; 72, 75) associated with a diaphragm plate (4a, 4b, 4c, 4d; 21; 61a, 62a, 63a, 64a) is provided, which gives a signal to the image-processing device (12), the signal corresponding to the position of the diaphragm plate (4a, 4b, 4c, 4d; 21; 61a, 62a, 63a, 64a) relative to the central ray (Z) of the X-ray beam, the image-processing device, during representation of a fluoroscopic image stored in the image memory (8), mixing a mark (20a, 20b, 20c, 20d; 76, 77, 78, 79) into the fluoroscopic image, the mark corresponding to the position of the diaphragm plate (4a, 4b, 4c, 4d; 21; 61a, 62a, 63a, 64a).

2. X-ray television installation according to claim 1, characterized in that with each diaphragm plate (4a, 4b, 4c, 4d) there is associated a position encoder (19a, 19b, 19c, 19d).

3. X-ray television installation according to claim 1, having a primary ray diaphragm (60) with diaphragm plates (61a, 62a or 63a, 64a) adjustable in pairs synchronously against one another, characterized in that a position encoder (75 or 72) is associated with one diaphragm plate (61a or 63a) of each pair of diaphragm plates (61a, 62a or 63a, 64a), and the image-processing device (12) generates the mark (78 or 79) corresponding to the other diaphragm plate (62a or 64a) of a pair.

4. X-ray television installation according to one of claims 1 to 3, characterized in that the mark (20a, 20b, 20c, 20d; 76, 77, 78, 79) is in each case formed by a line.

5. X-ray television installation according to claim 4, characterized in that the mark (20a, 20b, 20c, 20d; 76, 77, 78, 79) in its contour corresponds with the form of the gating edge of the corresponding diaphragm plate (4a, 4b, 4c, 4d; 61a, 61b, 61c, 61d).

6. X-ray television installation according to one of claims 1 to 5, characterized in that the position encoder (19a, 19b, 19c, 19d) is directly connected to the corresponding diaphragm plate (4a, 4b, 4c, 4d).

7. X-ray television installation according to one of claims 1 to 5, characterized in that the position encoder (59) is coupled with a motor (27) adjusting the corresponding diaphragm plate (21).

8. X-ray television installation according to one of claims 1 to 5, characterized in that the position encoder is formed by a stepping motor (75, 72) provided for the adjustment of the corresponding diaphragm plate (61a, 62a; 63a, 64a) and the signal of the position encoder is formed by the control signal for the stepping motor (75, 72).

**Revendications**

1. Installation de röntgentélévision servant à radiographier un objet à examiner (1), comportant un tube (2) à rayons X, un diaphragme (3,21,60) du rayonnement primaire, un amplificateur de brillance radiologique (5), une caméra de télévision (7) et un appareil de visualisation (13), et dans laquelle il est prévu, entre la caméra de télévision (7) et l'appareil de visualisation (13), une mémoire d'images (8) et un dispositif de traitement d'images (12), ce qui permet de représenter au choix une image radiologique enregistrée directement par la caméra de télévision (7) ou une image radiologique mémorisée dans la mémoire d'images (8), et dans laquelle le diaphragme du rayonnement primaire (3,22,60) comporte des plaques (4a,4b,4c,4d;21;61a,62a,63a,64a) qui peuvent être déplacées, pour collimater un faisceau de rayons X émis par le tube (2) à rayons X, caractérisé par le fait qu'il est prévu un capteur de distance (19a,19b,19c,19d; 59;72,75) qui est associé à une plaque (4a,4b,4c,4d; 21; 61a,62a,63a,64a) du diaphragme et qui envoie un signal correspondant à la position de la plaque (4a,4b,4c,4d; 21; 61a,62a,63a,64a) du diaphragme par rapport au rayon central (Z) du faisceau de rayons X, au dispositif de traitement d'images (12) qui, lors de la représentation d'une image radiologique mémorisée dans la mémoire d'images (8), insère dans l'image radiologique une marque (20a,20b,20c,20d; 76,77,78,79) qui correspond à la position de la plaque (4a,4b,4c,4d; 21; 61a,62a,63a,64a) du diaphragme.

2. Installation de röntgentélévision suivant la revendication 1, caractérisée par le fait qu'un capteur de distance (19a,19b,19c,19d) est associé à chaque plaque (4a,4b,4c,4d) du diaphragme.

3. Installation de röntgentélévision suivant la revendication 1, comportant un diaphragme du rayonnement primaire (60) comportant des plaques (61a,62a ou 63a,64a) déplaçables en synchronisme par couples les unes par rapport aux autres, caractérisée par le fait qu'un capteur de distance (75 ou 72) est associé respectivement à une plaque (61a ou 63a) d'un couple de plaques (61a,62a ou 63a,64a) du diaphragme, et que le dispositif de traitement d'images (12) produit la marque (78 ou 79) qui correspond à l'autre plaque (62a ou 64a) d'un couple de plaques du diaphragme.

4. Installation de röntgentélévision suivant l'une des revendications 1 à 3, caractérisée par le fait que la marque (20a,20b,20c,20d; 76,77,78,79) est formée par un trait.

5. Installation de röntgentélévision suivant la revendication 4, caractérisée par le fait que la configuration de la marque (20a,20b,20c,20d; 76,77,78,79) correspond à la forme du bord de la plaque correspondante (4a,4b,4c,4d; 61a,61b,61c,61d) du diaphragme.

6. Installation de röntgentélévision suivant l'une des revendications 1 à 5, caractérisée par le fait que le capteur de distance (19a,19b,19c,19d) est raccordé directement à la plaque correspondante (4a,4b,4c,4d) du diaphragme.

7. Installation de röntgentélévision suivant l'une des revendications 1 à 5, caractérisée par le fait que le capteur de distance (59) est accouplé à un moteur (27) qui déplace la plaque correspondante (21) du diaphragme.

8. Installation de röntgentélévision suivant l'une des revendications 1 à 5, caractérisée par le fait que le capteur de distance est formé par un moteur pas-à-pas (75,72), qui sert à déplacer la plaque correspondante (61a,62a; 63a,64a) du diaphragme, et que le signal du capteur de distance est formé par le signal de commande envoyé au moteur pas-à-pas (75,72).

FIG 1

EP 0 284 733 B1

FIG 2

FIG 3

FIG 4

FIG 5

EP 0 284 733 B1